# EUROPÄISCHE PATENTANMELDUNG

(11) **EP 2 145 612 A1**
(43) Veröffentlichungstag der Anmeldung: **20.01.2010**
(21) Anmeldenummer: 09008887.3
(22) Anmeldetag: 07.07.2009
(51) Int. Cl.: A61F 7/02

(54) **Passives Kalt-Warm-Element, sowie Verwendung von anorganischen Partikeln zu seiner Herstellung**

(30) Priorität: 14.07.2008 DE 102008032903
(71) Anmelder: Polycomp R. Reinders Produktionsgesellschaft mbH, 47546 Kalkar-Niedermörmter (DE)
(72) Erfinder: Reinders, Rudolf, 46459 Rees (DE)
(74) Vertreter: Walcher, Armin

(57) **Zusammenfassung**

Die vorliegende Erfindung betrifft ein passives Kalt-Warm-Element, umfassend einen flexiblen Behälter, der eine Dispersion enthält, die anorganische Partikel, wenigstens einen Gelbildner und/oder Viskositätsregler sowie wenigstens eine Flüssigphase umfasst, sowie weiterhin die Verwendung von anorganischen Partikeln zur Herstellung eines passiven Kalt-Warm-Elementes. Das erfindungsgemäße passive Kalt-Warm-Element wird vor der Verwendung abgekühlt bzw. erwärmt und führt nach Auflegen auf die Haut zu einer Abkühlung bzw. Erwärmung der entsprechenden Körperpartie.

## Beschreibung

Die vorliegende Erfindung betrifft ein passives Kalt-Warm-Element, umfassend einen flexiblen Behälter, der eine Dispersion, die anorganische Partikel, wenigstens einen Gelbildner und/oder Viskositätsregler sowie eine Flüssigphase umfasst, enthält sowie weiterhin die Verwendung von anorganischen Partikeln zur Herstellung des passiven Kalt-Warm-Elementes.

Die Wahrnehmung von Temperatur bzw. Temperaturänderungen wird durch Thermorezeptoren in der Haut vermittelt, wobei es sowohl Wärme- als auch Kälterezeptoren gibt. Kälte- und Wärmereize werden über Nervenfasern in das Gehirn weitergeleitet, welches abhängig von bestimmten Schwellenwerten einen Gegenregulationsprozess einleitet. Kälte führt beispielsweise zu einer Kontraktion der Blutgefäße, während Wärme diese erweitert. Wärme regt die Produktion von Schweiß an, Kälte wiederum löst Muskelzittern aus. Weil die Kälterezeptoren in der Haut eher oberflächlicher liegen als die Wärmerezeptoren, wird Kälte in der Regel schneller wahrgenommen, wobei unter Kälte im physikalischen Sinne ein Wärmeentzug zu verstehen ist.

Die Anwendungspalette der Thermotherapie (Kälte- und/oder Wärmetherapie) ist groß. So werden beispielsweise nach Sportverletzungen wie Verstauchungen, Zerrungen, Prellungen oder Quetschungen in der Akutphase kalte Wickel, Kältepackungen oder Kältesprays empfohlen, weil sich an den betroffenen Körperpartien die Gefäße verengen und somit der Flüssigkeitsaustritt aus Blut- und Lymphgefäßen und nachfolgend die Schwellung der betroffenen Körperpartie verringert wird. Zusätzlich werden die Reflexe des Nervensystems herabgesetzt und damit nimmt auch die Schmerzempfindung ab.

Durch Wärme wird dagegen der Stoffwechsel im Körper angeregt. Der Transport von Sauerstoff, Nährstoffen, Antikörpern und der Abstrom von Zerfallsprodukten, wie sie etwa bei Entzündungen der Gelenke oder bei Blutergüssen auftreten, werden mobilisiert. Die Wärmetherapie kann zum Beispiel bei Muskelverspannungen oder als Vorbereitung auf krankengymnastische Übungen eingesetzt werden.

Die Reaktion auf Kälte- und Wärmereize ist individuell und abhängig von verschiedensten Faktoren, wie beispielsweise dem Gewicht oder dem Alter. So lassen sich Symptome, wie etwa Kopfschmerzen oder Migräne mit einer Wärme- oder Kältetherapie lindern, abhängig vom jeweiligen Patienten.

Daneben lässt sich die Wärmeübertragung oder der Wärmeentzug auch auf andere Objekten anwenden, wie etwa die Erwärmung oder die Kühlung von Speisen und Getränken oder aber die Kühlung von temperaturempfindlichen Substanzen.

Seit jeher sind Hausmittel bekannt, die auf einer Wärmeübertragung oder einem Wärmeentzug beruhen. Diese Hausmittel umfassen unter anderem die Anwendung von feuchten Tüchern und Wickeln oder auch von mit Eiswürfeln gefüllten Beuteln. Ebenfalls bekannt sind Kissen, die mit partikelförmigen Naturprodukten, wie Dinkel oder Hirse gefüllt sind, und vor der Verwendung erwärmt oder abgekühlt werden. Insgesamt weisen diese Hausmittel aber eine Vielzahl von Nachteilen auf, beispielsweise im Hinblick auf ihre Anpassungsfähigkeit an unterschiedliche Formen oder ihre Wirkungsdauer.

Um diesen Nachteilen abzuhelfen, sind aus dem Stand der Technik Mittel und Vorrichtungen bekannt, welche die Erwärmung oder Abkühlung von Objekten erleichtern sollen.

Diese bestehen in der Regel aus einem flexiblen Beutel, der mit einer flüssigen Trägersubstanz gefüllt ist, die unter anderem einen Gefrierpunktserniedriger enthält, um ein Aushärten der Trägersubstanz bei der Lagerung im Eisfach oder Gefrierschrank zu verhindern. Vor der Anwendung werden die bekannten Mittel und Vorrichtungen vorerwärmt oder vorgekühlt.

So offenbart beispielsweise die US 3,885,403 eine Vorrichtung zur Verwendung als heiße oder kalte Kompresse. Die Kompresse umfasst eine robuste, flexible Hülle, die ein neutrales Gel enthält, welches seine gelartige Konsistenz über einen weiten Temperaturbereich beibehält. Das Gel besteht im wesentlichen aus Wasser, einem Verdickungsmittel und einem Gefrierpunktserniedriger.

Diese Kompresse weist einerseits den Nachteil auf, das sich diese nach dem Vorkühlen oder dem Vorerwärmen in kurzer Zeit an die Umgebungstemperatur angleicht und somit seine temperierende Wirkung verliert. Andererseits bildet diese wegen ihres geringen Eigengewichtes keinen vollflächigen Kontakt zwischen der Auflagefläche und der Haut aus, was zu einer unbefriedigenden Wärmeübertragung führt.

Die DE 295 21 567 U1 betrifft eine therapeutische Kalt-Warm-Kompresse. Die Kompresse umfasst einen flüssigkeitsdichten Beutel, der mit einer Trägersubstanz gefüllt ist, welche aus einem flüssigen Substrat und einem Kältemittel gemischt ist, wobei das Substrat im wesentlichen Humintorf ist.

Um die Wirkungsdauer der Wärmeübertragung zu erhöhen, enthält die Kalt-Warm-Kompresse zusätzlich flüssigen Humintorf, der organische Bestandteile in Form von zersetzten Pflanzenteilen enthält. Das nach Homogenisierung des Humintorfes erhaltene flüssige Substrat erhöht zwar die Wirkungsdauer der Wärmeübertragung gegenüber einer reinen gel-basierten Kompresse, wegen seines geringen Eigengewichtes bildet aber auch diese Kompresse keinen vollflächigen Kontakt zwischen der Auflagefläche und der Haut aus, was zu einer unbefriedigenden Wärmeübertragung führt.

Aus der WO 95/10252 ist eine Kompresse zur Kälte- und /oder Wärmebehandlung von Verletzungen bekannt, die in Form eines flexiblen Behälters vorliegt, der eine wässrige Lösung und diskrete Partikel eines quervernetzten, wasserabsorbierenden Polymers enthält. Die Kompresse enthält weiterhin ein Gefrierpunktserniedriger in Form eines Salzes und/oder eines Glykols.

Um die Wirkungsdauer der Wärmeübertragung zu erhöhen, enthält die Kompresse zusätzlich organische Partikel, bestehend aus einem quervernetzten, wasserabsorbierenden Polymer, die bei einer Vorerwärmung nicht schmelzen und somit sowohl zur Wärme- als auch zur Kältetherapie geeignet sind. Diese Kompresse erhöht zwar die Wirkungsdauer der Wärmeübertragung gegenüber einer reinen gel-basierten Kompresse, wegen seines geringen Eigengewichtes bildet aber auch diese Kompresse keinen vollflächigen Kontakt zwischen der Auflagefläche und der Haut aus, was zu einer unbefriedigenden Wärmeübertragung führt.

Die EP 0 123 949 A1 beschreibt die Verwendung eines verformbaren Beutels als Kälteträger. Der Beutel umfasst kleine Gelstücke, verpackt in einer flexiblen Hülle, wobei das Gel aus einer wässrigen Lösung oder Suspension eines Polyvinylalkohols und eines Gefrierpunktserniedrigers besteht. Das Gel wird in eine Gießform überführt, auf - 10°C oder tiefer abgekühlt und dann ohne Auftauen unter Vakuum teilweise dehydratisiert.

Dieser Kühlbeutel weist jedoch zum Einen den Nachteil auf, das er nur für eine Kältetherapie geeignet ist. Würde man nämlich den Gegenstand erhitzen, so käme es zum Schmelzen der Gelstücke, was nach erneuter Lagerung bei Temperaturen unterhalb des Gefrierpunktes zu einer gefrorenen, kompakten Masse führen würde. Zum Anderen bildet aber auch dieser Gegenstand wegen seines geringen Eigengewichtes ebenfalls keinen vollflächigen Kontakt zwischen der Auflagefläche und der Haut aus, was zu einer unbefriedigenden Wärmeübertragung führt.

Die Verwendung von anorganischen Partikeln zur Erwärmung oder Abkühlung ist aus dem Stand der Technik auch bekannt, jedoch werden in der Regel Partikel mit einem hohen Wasseraufnahmevermögen verwendet, wobei diese Partikel Flüssigkeiten absorbieren und/oder speichern. Vor der Anwendung werden die bekannten Mittel und Vorrichtungen vorerwärmt oder vorgekühlt.

So beschreibt die JP 9192158 eine Packung für Wärme- bzw. Kälteschutz bestehend aus einem verschlossenen Beutel, der ein poröses Keramikmaterial enthält, in dem Wasser absorbiert ist. Der verschlossene Beutel besteht aus einer wasserundurch-lässigen Folie. Das poröse Material weist eine Wasserabsorptionsrate von mehr als 30 % seines Eigengewichtes auf und wird aus der Gruppe ausgewählt, die aus Sepioliten, Attapulgiten, Perliten, Diatomerde, Cristobalit, Zeoliten, Vermiculit, Aktivkohle, aktivierter Tonerde und Mischungen der selben besteht. Die Größe der Partikel liegt vorzugsweise in einem Bereich bis 30 mm.

Aus der DE 692 31 988 T2 ist ein aus Partikeln bestehendes Agens für Erhitzung oder Kühlung bekannt, wobei ein poröses oder hohles Trägermaterial und eine auf Mikrowellen ansprechende flüssige Zusammensetzung verwendet wird, die in dem Trägermaterial zurückgehalten wird und Wasser enthält. Als Trägermaterial wird beispielsweise aktiviertes Aluminiumoxid, aktiviertes Siliziumdioxid oder ein Molekularsieb verwendet. Die bevorzugte Größe der verwendeten Partikel liegt im Bereich von 790 µm bis 3350 µm.

Die vorgenannten bekannten Verwendungen von anorganischen Partikeln zur Erwärmung oder Abkühlung weisen allesamt den Nachteil auf, dass die verwendeten Partikel aufgrund ihrer Absorptionseigenschaften eine poröse oder hohle Struktur aufweisen müssen, die für die verwendeten Flüssigkeiten zugänglich ist. Die Wärmespeicherfähigkeit von Feststoffen ist aber von ihrer spezifischen Dichte und ihrer spezifischen Wärmekapazität abhängig, wobei sich die spezifischen Dichte eines Feststoffes aus seiner Masse und dem Volumen einschließlich aller Hohlräume ergibt. Eine poröse oder hohle Struktur reduziert somit die spezifischen Dichte der verwendeten Partikel und führt mithin zu einer reduzierten Wärmespeicherfähigkeit. Gleichzeitig können die Partikel durch das herabsetzte Eigengewicht ein optimales Anschmiegen der Kompresse erst ab einer gewissen Partikelgröße gewährleisten, die dann aber wieder als störend auf der Haut empfunden werden kann.

Ein weiterer Nachteil einiger Mittel und Vorrichtungen des bekannten Stands der Technik besteht in der fehlenden Mikrowellenbeständigkeit. So führt etwa ein hoher Salzgehalt der Kompresse zu einer sehr schnellen lokalen Erhitzung bei Bestrahlung mit Mikrowellen, was zu einem Aufblähen bis hin zum Platzen der Kompresse führen kann.

Die bekannten Mittel und Vorrichtungen, die vor der Verwendung vorerwärmt oder vorgekühlt werden müssen, weisen zudem den Nachteil einer geringen Temperaturstabilität auf, d.h. sie gleichen sich nach dem Vorkühlen oder dem Vorerwärmen schnell der Umgebungstemperatur an und verlieren somit ihre temperierende Wirkung.

Aufgabe der vorliegenden Erfindung ist es, ein Kalt-Warm-Element bereitzustellen, das einen weitgehend vollflächigen Kontakt zwischen dem Kalt-Warm-Element und der zu behandelnden Körperpartie ausbildet und über einen längeren Zeitraum die vorgegebene Temperatur sowohl nach Vorerwärmung als auch nach Vorkühlung hält.

Eine weitere Aufgabe der vorliegenden Erfindung ist es, ein Kalt-Warm-Element bereitzustellen, das sich einfach und kostengünstig aus im wesentlichen ungiftigen, vorzugsweise ungiftigen, Bestandteilen herstellen lässt.

Die Aufgabe wird gelöst durch die Bereitstellung eines passiven Kalt-Warm-Elements, welches aus einem flexiblen Behälter besteht, der flüssigkeitsdicht ist und eine Dispersion enthält, die anorganische Partikel mit einer spezifischen Dichte aus einem Bereich von 1,7 bis 7,0 und einer Partikelgröße D₅₀ von maximal 100 µm, wenigstens einen Gelbildner und/oder Viskositätsregler sowie eine Flüssigphase umfasst, wobei der Anteil der anorganischen Partikel in einem Bereich von 20 bis 60 Gew.-%, der Anteil an Gelbildner und/oder Viskositätsregler in einem Bereich von 0,01 bis 10 Gew.-% und der Anteil an Flüssigphase in einem Bereich von 30 bis 79,99 Gew.-%, jeweils bezogen auf das Gesamtgewicht der Dispersion, liegt.

Bevorzugte Weiterbildungen der Erfindung sind in den Unteransprüchen angegeben.

Das erfindungsgemäße passive Kalt-Warm-Element wird vor der Verwendung abgekühlt bzw. erwärmt und führt nach Auflegen auf die Haut zu einer Abkühlung bzw. Erwärmung der entsprechenden Körperpartie.

Das erfindungsgemäße passive Kalt-Warm-Element behält überraschenderweise sowohl nach Vorerwärmung als auch nach Vorkühlung die wärmende bzw. kühlende Wirkung über einen längeren Zeitraum bei als herkömmliche, passive Kompressen. Unter einem passiven Element wird erfindungsgemäß ein Element, beispielsweise eine Kompresse, verstanden, die für die Kälte- oder Wärmeleistung keinen in dem passiven Kalt-Warm-Element ablaufenden endothermen oder exothermen Vorgang verwendet.

Darüberhinaus hat sich gezeigt, dass das erfindungsgemäße passive Kalt-Warm-Element nach mehrmaliger Verwendung, auch wiederholtem Kalt-Warm-Wechsel seine physikalische Konsistenz und temperierende Wirkung im wesentlichen nicht einbüßt.

Insgesamt behält das erfindungsgemäße passive Kalt-Warm-Element aufgrund der verwendeten anorganischen Partikel mit einer spezifischen Dichte aus einem Bereich von 1,7 bis 7,0 und einer Partikelgröße D₅₀ von maximal 100 µm die sowohl durch Vorerwärmung als auch durch Vorkühlung vorgegebene Temperatur über einen längeren Zeitraum bei als vergleichbare marktübliche, passive Kompressen. Dadurch kommt es insgesamt zu einer im wesentlichen verlängerten Wirkungsdauer des erfindungsgemäßen passiven Kalt-Warm-Elementes.

Das erfindungsgemäße passive Kalt-Warm-Element zeigt vorzugsweise ohne Lagerung im Kühlschrank über einen gewissen Zeitraum, der von der jeweiligen Größe des erfindungsgemäßen passiven Kalt-Warm-Elementes abhängig ist, eine als angenehm empfundene "Passivkälte", die der Umgebungstemperatur entspricht, worunter in diesem Zusammenhang ein Kälteempfinden verstanden wird, das bei vergleichbaren, nicht auf einem endothermen Vorgang beruhenden Kompressen nicht zu beobachten ist.

Dieser Vorteil des erfindungsgemäßen passiven Kalt-Warm-Elementes lässt sich vermutlich ebenfalls durch die thermischen Eigenschaften der verwendeten Dispersion erklären, ohne dabei an eine bestimmte Theorie gebunden zu sein.

Kälteempfinden entsteht in der Regel dann, wenn ein Gegenstand, der kälter ist als die aktuelle Körpertemperatur, im Normalfall 37°C, mit der Haut in Berührung gebracht wird, wie eingangs erläutert. Das erfindungsgemäße passive Kalt-Warm-Element weist ohne Vorerwärmung oder Vorkühlung eine Temperatur auf, die der Raumtemperatur entspricht und in der Regel bei 18 - 25°C liegt, und wird solange als kühlend empfunden, bis sich die Temperatur des erfindungsgemäßen passiven Kalt-Warm-Elementes an die Körpertemperatur angepasst hat.

Das erfindungsgemäße passive Kalt-Warm-Element kann bis zur Anpassung an die Körpertemperatur, in Abhängigkeit von der Zusammensetzung der verwendeten Dispersion, mehr Wärme vom Körper abführen als eine herkömmliche, passive Kompresse.

Die besonderen thermischen Eigenschaften des erfindungsgemäßen passiven Kalt-Warm-Elementes werden durch eine verbesserte Wärmeübertragung ergänzt.

Durch die Verwendung einer Dispersion von anorganischen Partikeln mit einer spezifischen Dichte aus einem Bereich von 1,7 bis 7,0 und einer Partikelgröße D₅₀ von maximal 100 µm kommt es zur Ausbildung eines weitgehend vollflächigen Kontaktes zwischen dem erfindungsgemäßen passiven Kalt-Warm-Element und dem zu behandelnden Körperbereich oder dem zu temperierenden Gegenstand in dem für eine Anwendung vorgesehenen Temperaturbereich.

Der für eine Anwendung auf einem Haut- oder Körperbereich vorgesehenen Temperaturbereich liegt üblicherweise in einem Bereich von -18°C und +60°C, weiter bevorzugt von -10°C bis +45°C, noch weiter bevorzugt von -5°C bis 40°C.

Bei der Anwendung an nichtbelebten Objekten, wie etwa bei der Erwärmung von Speisen und Getränken, kann der Temperaturbereich höher sein, wobei der Siedepunkt der wenigstens einen Flüssigphase nicht überschritten werden darf.

Beim Auflegen des erfindungsgemäßen passiven Kalt-Warm-Elementes auf die entsprechende Haut- oder Körperpartie kann es zur Ausbildung von Falten in dem verwendeten Beutel kommen, wodurch sich die Auflagefläche des passiven Kalt-Warm-Elementes auf der Haut signifikant reduzieren würde. Diese Faltenbildung wird aber durch das Eigengewicht des erfindungsgemäßen passiven Kalt-Warm-Elementes, das auf die erhöhte spezifische Dichte zurückzuführen ist, verhindert. Dadurch kommt es zu einem weitgehend vollflächigen Kontakt und damit verbundener optimaler Wärmeübertragung, ohne das der Benutzer zusätzlich das erfindungsgemäße passive Kalt-Warm-Element auf die entsprechende Hautpartie oder Körperbereich pressen muss. Dies ist besonders von Vorteil, wenn der Patient krank ist oder der thermisch zu behandelnde Körperbereich, beispielsweise das Schulterblatt, nicht oder nur schwer erreichbar ist.

Die übertragene Wärmemenge pro Zeiteinheit ist unter anderem von der Größe der Kontaktfläche abhängig, weshalb sich die übertragene oder entzogene Wärmemenge bei einem nicht vollflächigen Kontakt signifikant verringert. Dieser unerwünschte Effekt wird nach einer Lagerung bei Temperaturen unterhalb von 0°C durch eine gegebenenfalls auftretende Steifigkeit des verwendeten Behälters, beispielsweise Beutels verstärkt, so dass durch weitere Ausbildung von nicht vollflächigen Kontakten zwischen der Auflagefläche des erfindungsgemäßen passive Kalt-Warm-Elementes und der entsprechenden Hautpartie der erwünschte Kühlungseffekt weiter reduziert würde.

Es hat sich jedoch überraschender Weise gezeigt, dass das erfindungsgemäße passive Kalt-Warm-Element bei Temperaturen bis -18°C seine physikalische Konsistenz nicht signifikant verändert, zumindest aber plastisch leicht verformbar ist. Somit kann es auch nach längerer Lagerung bei -18°C sofort verwendet werden, und ein weitgehend vollflächiger Kontakt des erfindungsgemäßen passiven Kalt-Warm-Elementes mit der zu kühlende Hautpartie ist gewährleistet, ohne dass der Benutzer zusätzlich das erfindungsgemäße passive Kalt-Warm-Element auf die entsprechende Hautpartie pressen muss.

Der weitgehend vollflächige Kontakt des erfindungsgemäßen passiven Kalt-Warm-Elementes begründet sich in der spezifischen Dichte und der Teilchengröße der verwendeten anorganischen Partikel. Die spezifische Dichte und die Größe der anorganischen Partikel sind so eingestellt, dass die anorganischen Partikel in der verwendeten Dispersion vorzugsweise nicht bzw. nur verzögert sedimentieren. Dadurch wird eine optimale Wärmeübertragung gewährleistet und die für die Wärmeübertragung zur Verfügung stehende Kontaktfläche weitgehend nur durch die Abmessungen des erfindungsgemäßen passiven Kalt-Warm-Elementes begrenzt.

Der Anteil der anorganischen Partikel am Gesamtgewicht der Dispersion wird einerseits durch die Wirksamkeit der Wärmeübertragung und andererseits durch die spezifischen Dichte der anorganischen Partikel begrenzt.

Die besonderen Eigenschaften des erfindungsgemäßen passiven Kalt-Warm-Elementes werden durch einen Anteil der anorganischen Partikel am Gesamtgewicht der Dispersion erreicht, der in einem Bereich von 20 bis 60 Gew.-%, vorzugsweise von 30 bis 55 Gew.-% liegt.

Unterhalb eines Gewichtsanteils von 20 Gew.-% können die anorganischen Partikel keinen wesentlichen Einfluss mehr auf die thermischen Eigenschaften der Dispersion haben. Oberhalb eines Gewichtsanteils von 60 Gew.-% kann es dagegen schwierig sein, die Partikel aufgrund ihrer spezifischen Dichte gleichmäßig in Dispersion zu halten, so dass es zu einem Absinken der Partikel kommt. Dies führt wiederum dazu, dass das Kalt-Warm-Element bei Temperaturen unterhalb des Schmelzpunktes der wenigstens einen Flüssigphase seine physikalische Konsistenz verliert und gegebenenfalls aushärtet.

Die Wärmespeicherfähigkeit von Feststoffen ist von ihrer spezifischen Dichte und ihrer spezifischen Wärmekapazität abhängig, wobei sich die spezifischen Dichte eines Feststoffes aus seiner Masse und dem Volumen einschließlich aller Hohlräume ergibt. Eine poröse oder hohle Struktur reduziert die spezifischen Dichte der Partikel und führt mithin zu einer reduzierten Wärmespeicherfähigkeit. Im Gegensatz dazu weisen die erfindungsgemäßen anorganischen Partikel eine geringe Porosität auf, was sich in ihrer geringen Wasserabsorption wiederspiegelt.

Auf Grund ihrer spezifischen Dichte im Bereich von 1,7 bis 7,0 besitzen die anorganischen Partikel ein ausreichendes Eigengewicht, um ein optimales Anschmiegen der Kompresse bei einer Partikelgröße D₅₀ von maximal 100 µm zu gewährleisten. Diese Partikelgröße wird beim Auflegen des Kalt-Warm-Elementes auf eine Hautpartie nicht als störend empfunden.

Die verwendeten anorganischen Partikel weisen eine spezifische Dichte im Bereich von 1,7 bis 7,0, vorzugsweise von 2,2 bis 6,6, weiter bevorzugt von 2,5 bis 4,0 auf. Die Dichte der anorganischen Partikel wird vorzugsweise gemäß DIN ISO 787/10 bestimmt.

Bei der Verwendung von anorganischen Partikeln mit einer geringeren spezifischen Dichte kann entsprechend ein höherer Partikelanteil in der Dispersion verwendeten werden, ohne das es zum Absinken der Partikel und der damit verbundenen Nachteile kommt. Dabei kann es bei einer geringeren spezifischen Dichte zu einer verminderten Wärmespeicherfähigkeit der anorganischen Partikel kommen, der durch eine Erhöhung des Partikelanteils in der Dispersion aufgewogen werden kann.

Bei einer bevorzugten Ausführungsform der Erfindung liegen die anorganischen Partikel mit einer spezifischen Dichte aus einem Bereich von 1,7 bis 7,0 und einer Partikelgröße D₅₀ von maximal 100 µm in einer Dispersion als dispergierte Phase vor.

Die Größe der anorganischen Partikel hat ebenfalls einen wichtigen Einfluss auf die Eigenschaften der Dispersion. Sind die verwendeten Partikel zu klein, so ist ihr Einfluss auf das thermische und physikalische Verhalten der Dispersion gering.

Bei zu großen Partikeln entsteht demgegenüber einerseits das Problem, die Partikel gleichmäßig in Dispersion zu halten, andererseits kann es beim Auflegen des Kalt-Warm-Elementes auf die Haut zu einem störenden Fremdkörperempfinden kommen.

Die Größe der anorganischen Partikel, die in dem erfindungsgemäßen Kalt-Warm-Element verwendet werden, wird vorzugsweise mittels Lasergranulometrie bestimmt, wobei es sich um ein Laserbeugungsverfahren handelt, bei dem die Größe der Partikel aus der Beugung des Laserlichts ermittelt wird. Vorzugsweise wird die Lasergranulometrie mit dem Gerät Helos der Fa. Sympatec, Clausthal-Zellerfeld, Deutschland, gemäß Herstellerangaben durchgeführt.

Bei der Bestimmung der Größe der anorganischen Partikel durch Lasergranulometrie wird anhand der Beugung eines Laserstrahls an den Partikelrändern auf das Volumen der Teilchen geschlossen, wobei davon ausgegangen wird, dass die Teilchen annähernd kugelförmig sind. Somit beziehen sich die ermittelten Durchmesser stets auf den über alle Raumrichtungen gemittelte Äquivalentkugeldurchmesser, unabhängig von der tatsächlichen Form der anorganischen Partikel.

Ermittelt wird eine Größenverteilung, die in Form eines Volumenmittels, bezogen auf den Äquivalentkugeldurchmesser, berechnet wird. Diese volumengemittelte Größenverteilung kann u.a. als Summenhäufigkeitsverteilung dargestellt werden. Die Summenhäufigkeitsverteilung wiederum wird meist vereinfachend durch bestimmte Kennwerte charakterisiert, z. B. den D₅₀- oder D₉₀-Wert. Unter einem D₉₀-Wert wird verstanden, dass 90 % aller Partikel gleich diesem Wert sind oder unter dem angegebenen Wert liegen. Anders ausgedrückt, liegen 10 % aller Partikel oberhalb des angegebenen Wertes. Bei einem D₅₀-Wert liegen 50 % aller Partikel gleich oder unter und 50 % aller Partikel oberhalb des angegebenen Wertes.

Die verwendeten anorganischen Partikel weisen vorzugsweise ein D₉₈ von maximal 100 µm, bevorzugt von maximal 60 µm, weiter bevorzugt von maximal 30 µm auf. Die mittlere Partikelgröße D₅₀ der anorganischen Partikel liegt vorzugsweise in einem Bereich von 0,1 bis 50 µm, bevorzugt von 1 bis 20 µm, weiter bevorzugt von 1 bis 10 µm.

Es hat sich gezeigt, dass die spezifische Dichte und die Größe der erfindungsgemäß zu verwendenden anorganischen Partikel ausreicht, um die Ausbildung eines weitgehend vollflächigen Kontaktes zwischen der Auflagefläche des passiven Kalt-Warm-Elementes und der Haut- bzw. Körperpartie zu gewährleisten.

Darüber hinaus hat auch die Kompaktheit der anorganischen Partikel einen Einfluss auf die thermischen Eigenschaften und das Gewicht des erfindungsgemäßen passiven Kalt-Warm-Elementes. Die Kompaktheit der verwendeten anorganischen Partikel spiegelt sich in der Wasserabsorption wieder. Die Bestimmung der Wasserabsorption erfolgt vorzugsweise wie die Bestimmung der Ölabsorption nach DIN ISO 787/5, wobei anstelle des Leinöls destilliertes oder deionisiertes Wasser eingesetzt wird. Die Vorrichtungen sind die gleichen wie bei der Bestimmung der Ölabsorption.

Bevorzugte anorganische Partikel weisen eine Wasserabsorption, bezogen auf das Gesamtgewicht der trockenen anorganischen Partikel, von weniger als 30 Gew.-%, besonders bevorzugt von weniger als 25 Gew.-% auf. Weiterhin ist bevorzugt, dass die Wasserabsorption bei weniger als 20 Gew.-%, noch weiter bevorzugt bei weniger als 10 Gew.-%, liegt, wobei sich die Gew.-% jeweils auf das Gewicht der trockenen anorganischen Partikel beziehen.

Bei einer bevorzugten Ausführungsform ist das erfindungsgemäße passive Kalt-Warm-Element in der Mikrowelle erhitzbar. Bevorzugte anorganische Partikel sind deshalb in der Lage, Mikrowellen zu absorbieren. Ferner überschreitet vorzugsweise die Löslichkeit der verwendeten Partikel in der wenigstens einen Flüssigphase in dem für eine Anwendung vorgesehenen Temperaturbereich einen bestimmten Grenzwert nicht.

Bevorzugte anorganische Partikel weisen eine Löslichkeit in Wasser, die vorzugsweise gemäß DIN ISO 787/8 bestimmt wird, bei 20°C von weniger als 2,0 g/l, besonders bevorzugt von weniger als 0,5 g/l auf.

Vorzugsweise sind die verwendeten Partikel in der wenigstens einen Flüssigphase in einem Temperaturbereich von bis zu 100°C schwer oder praktisch unlöslich.

Es ist bekannt, dass Mikrowellen an elektrisch stark leitenden Oberflächen reflektiert werden und die Energie des Mikrowellenfeldes daher nicht oder nur teilweise in das Material eindringen kann. Dies führt beispielsweise bei der Bestrahlung von sehr salzhaltigen Kompressen mit Mikrowellen zu einer ungleichmäßig stärkeren Erhitzung der Kompressenoberfläche gegenüber dem Kompressenkern. Außerdem kann es, abhängig vom Gehalt an gelösten Ionen, zu einem schlagartigen, oft unkontrollierten Erhitzungsvorgang kommen, der ein Aufblähen bis hin zum Platzen einer Kompresse bewirkt.

Bevorzugte anorganische Partikel dissoziieren in der wenigstens einen Flüssigphase nicht oder nur unwesentlich, wodurch eine Erhitzung des erfindungsgemäßen Kalt- Warm-Elementes in der Mikrowelle ermöglicht wird.

Vorzugsweise reagieren die verwendeten Partikel im wesentlichen nicht mit dem Gelbildner und/oder Viskositätsregler oder der wenigstens einen Flüssigphase. Dadurch wird eine langanhaltende Funktion des erfindungsgemäßen passiven Kalt-Warm-Elementes gewährleistet.

Gemäß einer bevorzugten Ausführungsform werden anorganische Partikel verwendet, die einerseits durch nasse oder trockene Vermahlung von natürlich vorkommendem Gestein erhalten werden. Es hat sich überraschenderweise gezeigt, dass Gesteinsmehl oder Gesteinspulver für die Zwecke der vorliegenden Erfindung sehr geeignet ist. Äußerst vorteilhaft ist dabei, dass sie kostengünstig und in ausreichender Menge verfügbar sind sowie auch im Fall einer eventuellen Undichtigkeit des erfindungsgemäßen passiven Kalt-Warm-Elementes im wesentlichen keine Gesundheitsgefahr für den Verwender darstellen. Unter der Bezeichnung Gestein wird erfindungsgemäß eine feste, in der Regel mikroskopisch heterogene Vereinigung von Mineralen, Gesteinsbruchstücken, Gläsern oder Rückständen von Organismen mit einem weitgehend konstanten Mischungsverhältnis dieser Bestandteile zueinander verstanden.

Selbstverständlich können die anorganischen Partikel auch synthetisch, beispielsweise durch Ausfällung oder Auskristallisierung, hergestellt werden und durch nasse oder trockene Vermahlung in der entsprechenden Partikelgröße erhalten werden.

Die anorganischen Partikel können durch Beschichtung chemisch und/oder physikalisch verändert werden, wobei dies vorzugsweise während des Vermahlens oder nach dem Vermahlen durch eine Wärmebehandlung geschieht. Geeignete Beschichtungen sind beispielsweise Magnesiumstearat oder -oleat, ohne auf diese begrenzt zu sein. Die Morphologie der anorganischen Partikel kann durch den Mahlprozess auch verändert werden, indem verschiedene Mahltechniken verwendet werden.

Entscheidend für die Funktion des erfindungsgemäßen passiven Kalt-Warm-Elementes ist insbesondere die Größe und die spezifische Dichte der verwendeten anorganischen Partikel.

Gemäß einer bevorzugten Ausführungsform können die anorganischen Partikel natürlich vorkommendes Gestein enthalten oder daraus bestehen, das vorzugsweise aus der Gruppe ausgewählt wird, die aus Basalt, Sandstein, Kalksandstein, Kalkstein, Schiefer, Tonschiefer, Serpentinen, Steatit, Talk, Löss, Ton, Tonerden, Kaolin, Mergel und Mischungen derselben besteht.

Gemäß einer weiteren bevorzugten Ausführungsform der Erfindung können die anorganischen Partikel Metallcarbonat enthalten oder aus Metallcarbonat bestehen, wobei das Metall vorzugsweise aus der Gruppe, die Magnesium, Calcium, Strontium, Barium, Mangan, Eisen, Cobalt, Nickel, Kupfer, Zink, Silber, Cadmium, Bismut, Blei und Mischungen derselben besteht, ausgewählt wird. Es können auch weitere Metalle in Carbonaten mit den vorgenannten Metallen vorliegen, beispielsweise in Form von Verunreinigungen.

Bevorzugte Metallcarbonate weisen eine Löslichkeit in Wasser, die vorzugsweise gemäß DIN ISO 787/8 bestimmt wird, bei 20°C von weniger als 2,0 g/l, besonders bevorzugt von weniger als 0,5 g/l, auf.

Gemäß einer weiteren bevorzugten Ausführungsform der Erfindung können die anorganischen Partikel natürlich vorkommendes Calciumcarbonat enthalten oder daraus bestehen, das vorzugsweise einen Calciumcarbonat-Anteil, bezogen auf das Gesamtgewicht der anorganischen Partikel, von 50 bis 100 Gew.-% aufweist.

Natürlich vorkommendes Calciumcarbonat wird vorzugsweise aus der Gruppe, die aus Calcit, Korallenkalk, Muschelkalk, Dolomit, Kalkstein, Kalkspat, Aragonit, Marmor, Travertin, Kreide und Mischungen davon besteht, ausgewählt.

Bei einer ganz besonders bevorzugten Ausführungsform können die anorganischen Partikel aus Rügen-Kreide bestehen oder Rügen-Kreide enthalten. Die Rügen-Kreide weist vorzugsweise einen Calciumcarbonat-Anteil, bezogen auf das Gesamtgewicht der anorganischen Partikel, von 50 % bis 100 Gew.-%, bevorzugt von 70 % bis 100 Gew.-%, besonders bevorzugt von 90 % bis 99,8 Gew.-% auf.

Weiterhin ist bevorzugt, dass die Rügen-Kreide, bezogen auf das Gesamtgewicht der anorganischen Partikel, einen Magnesiumcarbonat-Anteil im Bereich von 0,2 bis 2,5 Gew.-% aufweist. Vorzugsweise weist die Rügen-Kreide einen in Salzsäure unlösliche Anteil, der vorzugsweise nach DIN 55918 bestimmt wird, von weniger als 3 Gew.-%, bevorzugt weniger als 1,5 Gew.-%, jeweils bezogen auf das Gesamtgewicht der anorganischen Partikel, auf.

Es hat sich überraschend gezeigt, dass Rügenkreide bei der Lösung der der Erfindung zugrundeliegenden Aufgabe besonders geeignet ist.

Gemäß einer weiteren bevorzugten Ausführungsform der Erfindung können die anorganischen Partikel präzipitiertes Calciumcarbonat, vorzugsweise mit einem Calciumcarbonat-Anteil von 50 % bis 100 Gew.-%, bevorzugt von 70 % bis 100 Gew.-%, jeweils bezogen auf das Gesamtgewicht der anorganischen Partikel, enthalten oder daraus bestehen.

Sowohl präzipitiertes als auch natürlich vorkommendes Calciumcarbonat kann in amorpher Form vorliegen und/oder aus den kristallinen Modifikationen Calcit, Aragonit oder Vaterit und/oder Mischungen derselben bestehen. Vorzugsweise liegen nach Vermahlung die kristallinen Modifikationen als Einzelkristalle und/oder deren Bruchstücke vor. In natürlich vorkommendem Calciumcarbonat können weiterhin fossile Überreste von maritimen Lebewesen vorkommen.

Die anorganischen Partikel werden durch wenigstens einen Gelbildner und/oder Viskositätsregler dispergiert. Dabei wird der Anteil des wenigstens einen Gelbildners und/oder Viskositätsreglers vorzugsweise so gewählt, dass einerseits die anorganischen Partikel gleichmäßig in Dispersion gehalten werden und dass andererseits das erfindungsgemäße passive Kalt-Warm-Element seine Verformbarkeit nicht verliert.

Der Gewichtsanteil des Gelbildners und/oder Viskositätsreglers liegt in einem Bereich von 0,01 bis 10 Gew.-%, vorzugsweise von 0,05 bis 5 Gew.-%, weiter bevorzugt von 0,1 bis 2,5 Gew.-%, jeweils bezogen auf das Gesamtgewicht der Dispersion.

Vorzugsweise weist die Dispersion nicht-newtonsche Eigenschaften auf, d.h. die Viskosität der Dispersion ändert sich unter Einwirkung von Scherkräften. Weiterhin ist bevorzugt das die Dispersion thixotrope Eigenschaften aufweist, d.h. die Viskosität verringert sich unter Einwirkung von Scherkräften. Dadurch lässt sich die Form des erfindungsgemäße passive Kalt-Warm-Element mit geringem Druck durch den Benutzer an verschiedene Körperpartien, wie etwa das Knie, den Ellenbogen oder das Fußgelenk anpassen.

Nach Aussetzung der Scherkräfte baut sich die Ausgangsviskosität wieder auf und das erfindungsgemäße passive Kalt-Warm-Element bildet einen weitgehend vollflächigen Kontakt zwischen dem Kalt-Warm-Element und der Haut- bzw. Körperpartie auf, aufgrund der Verwendung einer Dispersion von anorganischen Partikeln mit einer spezifischen Dichte aus einem Bereich von 1,7 bis 7,0 und einer Partikelgröße D₅₀ von maximal 100 µm.

Der Gelbildner und/oder Viskositätsregler wird bevorzugt aus der Gruppe, die aus Agar, Alginsäure, Alginate, Gummi arabicum, Traganth, Galactan, Carobgummi, Polyosen, Guarkernmehl, Karayagummi, Carrageen, Pektin, Quittensamenmehl, Casein, Gelatine, Kollagen, Albumin, Stärke, vorzugsweise aus Reis, Mais, Kartoffel, Weizen, modifizierte Stärke, Tarakernmehl, Johannisbrotbaumkernmehl, Xanthan, Dextran, Gellan, Furcellaran, Pullulan, Phyllophoran, Chitin, Chitosan, Polyimine, Polyamide, Vinylpolymere, Polyoxyethylen-Polyoxypropylen-Copolymere, Polyacryl- und Polymethacrylverbindungen, Polycarbonsäuren, Polyoxyethylenpolymere, Polyether wie Polyethylenglycole, Cellulosepolymere wie Carboxymethylcellulose, Hydroxymethylcellulose, Hydroxypropylcellulose, Hydroxypropylmethylcellulose, Methylcellulose, Methylethylcellulose, Hyaluronsäure und Mischungen derselben besteht, ausgewählt.

Der Gewichtsanteil der Flüssigphase liegt, bezogen auf das Gesamtgewicht der Dispersion, in einem Bereich von 30 bis 79,99 Gew.-%. Vorzugsweise liegt der Anteil der Flüssigphase in einem Bereich von 40 bis 70 Gew.-%, weiter bevorzugt von 50 bis 60 Gew.-%, jeweils bezogen auf das Gesamtgewicht der Dispersion.

Erfindungsgemäß wird unter einer Flüssigphase eine Substanz verstanden, die unter Standardbedingungen (Temperatur: 25°C, Luftdruck: 1013 mbar) einen flüssigen Aggregatzustand aufweist. Vorzugsweise besteht die Flüssigphase aus einer Flüssigkeit oder einem homogenen Gemisch von Flüssigkeiten. Weiterhin ist bevorzugt, wenn die Flüssigphase im wesentlichen nicht mit den verwendeten anorganischen Partikeln reagiert.

Gemäß einer Variante der Erfindung kann eine Mischung aus Wasser und wenigstens einem Alkohol als Flüssigphase verwendet werden. Vorzugsweise wird ein Alkanol mit ein bis fünf C-Atomen verwendet, das ein bis drei funktionelle OH-Gruppen enthält, wobei weiter bevorzugt das Alkanol aus der Gruppe, die aus Ethanol, Propan-1-ol, Propan-2-ol, Ethan-1,2-diol, Propan-1,2-diol, Propan-1,3-diol und Propan-1,2,3-triol besteht, ausgewählt wird.

Vorzugsweise wird Wasser als Flüssigphase verwendet.

Das erfindungsgemäße passive Kalt-Warm-Element kann aber auch mehr als eine Flüssigphase enthalten, beispielsweise eine stabile Emulsion mehrerer Flüssigphasen, die mittels wenigstens eines Emulgators und/oder Tensids hergestellt und homogenisiert und/oder stabilisiert wurde.

Der flüssigkeitsdichte Beutel ist in dem für eine Anwendung vorgesehenen Temperaturbereich vorzugsweise reiß- und stichfest. Erfindungsgemäß enthält der flüssigkeitsdichte Beutel eine Dispersion von anorganischen Partikeln mit einer spezifischen Dichte aus einem Bereich von 1,7 bis 7,0 und einer Partikelgröße D₅₀ von maximal 100 µm, wenigstens einen Gelbildner und/oder Viskositätsregler sowie eine Flüssigphase. Bei einer besonderen Ausführungsform liegt diese Dispersion luftblasenfrei in dem flüssigkeitsdichten Beutel vor. Dadurch wird die Wärmeübertragung des erfindungsgemäßen passiven Kalt-Warm-Elementes weiter erhöht. Darüber hinaus wird bei einer Erwärmung des erfindungsgemäßen passiven Kalt-Warm-Elementes innerhalb des für eine Anwendung vorgesehenen Temperaturbereiches ein Ausdehnen oder gar Platzen des flüssigkeitsdichten Beutels infolge eines Luftüberdruckes im wesentlichen verhindert.

Vorzugsweise erlaubt der flüssigkeitsdichte Beutel ein optimales Anschmiegen an einen Untergrund, beispielsweise eine Wade, Wange etc., und eine ausreichende Wärmeübertragung des erfindungsgemäßen passiven Kalt-Warm-Elementes.

Der flexible Behälter ist vorzugsweise aus einem Thermoplasten, beispielsweise aus Polyamid/Polyethylen-Folie, Polyamid-Folie, Polypropylen-Folie, Ethylen-Vinyl-Acetat-Folie oder dergleichen hergestellt. Der flexible Behälter kann dabei sowohl einschichtig als auch mehrschichtig aufgebaut sein. Die Dicke des Folienmaterials kann in Abhängigkeit von der Größe des flexiblen Behälters und des Gewichtes der Dispersion eingestellt werden.

Der flexible Behälter ist vorzugsweise aus einer Kammer aufgebaut, was eine einfache und kostengünstige Herstellung erlaubt.

Selbstverständlich kann der flexible Behälter im einfachsten Fall auch aus verschweißten Folien hergestellt werden. Der Fachmann wählt dann das Material und die Materialdicke in Abhängigkeit von der Größe des erfindungsgemäßen passiven Kalt-Warm-Elementes und des Gewichtes der Dispersion.

Der flexible Behälter kann transparent oder matt sein. Ebenso kann der flexible Behälter farblos oder farbig sein.

Die Oberfläche des flexiblen Behälters kann glatt oder strukturiert sein. Die Oberfläche des flexiblen Behälters ist vorzugsweise geprägt, bevorzugt mikrogeprägt, so dass die Oberfläche Erhebungen und Vertiefungen aufweist, wobei die Erhebungen und Vertiefungen vorzugsweise im Mikrometerbereich liegen. Bei der Anwendung auf der Haut wird damit ein Ankleben des Kalt-Warm-Elementes an der Haut verhindert.

Bei einer bevorzugten Ausführungsform ist der flexible Behälter verschließbar. Der Verschluss kann aus einem Schraubverschluss, einem Stopfen oder Ähnlichem bestehen. Der verschließbare, flexible Behälter enthält anorganische Partikel mit einer spezifischen Dichte aus einem Bereich von 1,7 bis 7,0 und einer Partikelgröße D₅₀ von maximal 100 µm sowie wenigstens einen Gelbildner und/oder Viskositätsregler, wobei das Gewichtsverhältnis von anorganischen Partikeln zu Gelbildner und/oder Viskositätsregler einen Bereich zwischen 2 und 6000 umfasst. Diese Ausführungsform der Erfindung ermöglicht einen einfachen und kostengünstigen Transport des erfindungsgemäßen passiven Kalt-Warm-Elementes.

Die Zugabe der wenigstens einen Flüssigphase, vorzugsweise Wasser, erfolgt durch den Anwender.

Nach Zugabe von Flüssigkeit in den verschließbaren, flexiblen Behälter liegt eine Dispersion vor, die anorganische Partikel, wenigstens einen Gelbildner und/oder Viskositätsregler sowie wenigstens eine Flüssigphase enthält, wobei der Anteil der anorganischen Partikel in einem Bereich von 20 bis 60 Gew.-%, der Anteil an Gelbildner und/oder Viskositätsregler in einem Bereich von 0,01 bis 10 Gew.-% und der Anteil an Flüssigphase in einem Bereich von 30 bis 79,99 Gew.-%, jeweils bezogen auf das Gesamtgewicht der Dispersion, liegt

Das erfindungsgemäße passive Kalt-Warm-Element kann weiterhin einen oder mehrere Neutralisatoren enthalten. Vorzugsweise handelt es sich um NaOH oder KOH oder alternativ um aliphatische und/oder zyklische Amine, wie etwa Monoethanolamin, Triethanolamin, Diisopropanolamin und Cyclohexylamin oder dergleichen.

Im Rahmen einer bevorzugten Ausführungsform der vorliegenden Erfindung liegt der pH-Wert der Dispersion, vorzugsweise bestimmt gemäß DIN ISO 787/9, in einem Bereich von 5 bis 8,5, vorzugsweise in einem Bereich von 6 bis 8 und insbesondere in einem Bereich von etwa 6,5 bis etwa 7,5.

Das erfindungsgemäße passive Kalt-Warm-Element kann weiterhin Farbstoffe enthalten, die beispielsweise in Abhängigkeit von der Temperatur ihre Farbe ändern und so dem Benutzer die Temperatur des erfindungsgemäßen passiven Kalt-Warm-Elementes anzeigen.

Weiterhin kann das erfindungsgemäße passive Kalt-Warm-Element antimikrobielle Substanzen, Konservierungsstoffe, UV-Stabilisatoren und dergleichen enthalten.

Die Erfindung wird nachfolgend durch Beispiele erläutert, ohne hierauf beschränkt zu sein.

### Beispiel 1:

In eine wässrige Lösung mit 0,9 Gew.-% Hydroxymethylcellulose wurden bei 25°C unter ständigem Rühren 45,0 Gew.-% Dolomitpuder 20 µm (Schöndorfer GmbH, Schneizlreuth), jeweils bezogen auf das Gesamtgewicht der Dispersion, dispergiert. Das Dolomitpuder 20 µm weist gemäß Herstellerangabe einen Calciumcarbonat-Anteil von 54,8 Gew.-%, einen Magnesiumcarbonat-Anteil von 45 Gew.-%, jeweils bezogen auf das Gesamtgewicht der anorganischen Partikel, und eine Partikelgröße D₅₀ von 6,7 µm auf. 425 ml dieser Dispersion wurden in einen handelsüblichen Polyethylenbeutel mit den Abmessungen 12 cm mal 29 cm gefüllt und luftblasenfrei verschweißt.

### Beispiel 2:

In eine wässrige Lösung mit 0,75 Gew.-% Xanthan wurden bei 25°C unter ständigem Rühren 50,0 Gew.-% micral® 10 (Reverté GmbH, Köln), jeweils bezogen auf das Gesamtgewicht der Dispersion, dispergiert. micral® 10 weist gemäß Herstellerangabe einen Calciumcarbonat-Anteil von 98,84 Gew.-%, bezogen auf das Gesamtgewicht der anorganischen Partikel, und eine Partikelgröße D₅₀ von 4,8 µm auf. 440 ml dieser Dispersion wurden in einen handelsüblichen Polyethylenbeutel mit den Abmessungen 12 cm mal 29 cm gefüllt und luftblasenfrei verschweißt.

### Beispiel 3:

In eine wässrige Lösung mit 1,25 Gew.-% Polyethylenglykol 800 wurden bei 25°C unter ständigem Rühren 40,0 Gew.-% Rügener Schlämmkreide 32 (Vereinigte Kreidewerke Dammann KG, Sölde), jeweils bezogen auf das Gesamtgewicht der Dispersion, dispergiert. Die Schlämmkreide weist gemäß Herstellerangabe einen Calciumcarbonat-Anteil von 97,5 Gew.-%, bezogen auf das Gesamtgewicht der anorganischen Partikel, und eine Partikelgröße D₅₀ von 2 µm auf. 428 ml dieser Dispersion wurden in einen handelsüblichen Polyethylenbeutel mit den Abmessungen 12 cm mal 29 cm gefüllt und luftblasenfrei verschweißt.

### Beispiel 4:

In eine wässrige Lösung mit 0,6 Gew.-% Agar wurden bei 25°C unter ständigem Rühren 38,0 Gew.-% Marmormehl mittel (Kremer Pigmente GmbH & Co. KG, Aichstetten), jeweils bezogen auf das Gesamtgewicht der Dispersion, dispergiert. Das Marmormehl mittel weist gemäß Herstellerangabe einen Calciumcarbonat-Anteil von 99,2 Gew.-%, bezogen auf das Gesamtgewicht der anorganischen Partikel, und eine Partikelgröße D₉₈ von 60 µm auf. 440 ml dieser Dispersion wurden in einen handelsüblichen Polyethylenbeutel mit den Abmessungen 12 cm mal 29 cm gefüllt und luftblasenfrei verschweißt.

## Patentansprüche

1. Passives Kalt-Warm-Element, umfassend einen flexiblen Behälter,
**dadurch gekennzeichnet,**
**dass** der flexible Behälter flüssigkeitsdicht ist und eine Dispersion enthält, die anorganische Partikel mit einer spezifischen Dichte aus einem Bereich von 1,7 bis 7,0 und einer Partikelgröße D₅₀ von maximal 100 µm, wenigstens einen Gelbildner und/oder Viskositätsregler sowie eine Flüssigphase umfasst, wobei der Anteil der anorganischen Partikel in einem Bereich von 20 bis 60 Gew.-%, der Anteil an Gelbildner und/oder Viskositätsregler in einem Bereich von 0,01 bis 10 Gew.-% und der Anteil an Flüssigphase in einem Bereich von 30 bis 79,99 Gew.-%, jeweils bezogen auf das Gesamtgewicht der Dispersion, liegt.

2. Passives Kalt-Warm-Element, umfassend einen flexiblen Behälter,
**dadurch gekennzeichnet,**
**dass** der flexible Behälter verschließbar ist und anorganische Partikel mit einer spezifischen Dichte aus einem Bereich von 1,7 bis 7,0 und einer Partikelgröße D₅₀ von maximal 100 µm sowie wenigstens einen Gelbildner und/oder Viskositätsregler enthält, wobei das Gewichtsverhältnis von anorganischen Partikeln zu Gelbildner und/oder Viskositätsregler in einem Bereich zwischen 2 und 6000 liegt.

3. Passives Kalt-Warm-Element nach Anspruch 2,
**dadurch gekennzeichnet,**
**dass** nach Zugabe von Flüssigkeit eine Dispersion vorliegt, die anorganische Partikel, wenigstens einen Gelbildner und/oder Viskositätsregler sowie wenigstens eine Flüssigphase enthält, wobei der Anteil der anorganischen Partikel in einem Bereich von 20 bis 60 Gew.-%, der Anteil an Gelbildner und/oder Viskositätsregler in einem Bereich von 0,01 bis 10 Gew.-% und der Anteil an Flüssigphase in einem Bereich von 30 bis 79,99 Gew.-%, jeweils bezogen auf das Gesamtgewicht der Dispersion, liegt.

4. Passives Kalt-Warm-Element nach einem der vorgenannten Ansprüche,
**dadurch gekennzeichnet,**
**dass** die anorganischen Partikel eine Partikelgröße D₅₀ aus einem Bereich von 0,1 bis 50 µm, bevorzugt von 1 bis 20 µm, aufweisen.

5. Passives Kalt-Warm-Element nach einem der vorgenannten Ansprüche,
**dadurch gekennzeichnet,**
**dass** die anorganischen Partikel ein D₉₈ von kleiner oder gleich 100 µm, bevorzugt von kleiner oder gleich 60 µm, aufweisen.

6. Passives Kalt-Warm-Element nach einem der vorgenannten Ansprüche,
**dadurch gekennzeichnet,**
**dass** die anorganischen Partikel natürlich vorkommendes Gestein, das vorzugsweise aus der Gruppe, die aus Basalt, Sandstein, Kalksandstein, Kalkstein, Schiefer, Tonschiefer, Serpentinen, Steatit, Talk, Löss, Ton, Tonerden, Kaolin, Mergel und Mischungen derselben besteht, ausgewählt wird, enthalten oder daraus bestehen.

7. Passives Kalt-Warm-Element nach einem der vorgenannten Ansprüche,
**dadurch gekennzeichnet,**
**dass** die anorganischen Partikel ein Metallcarbonat, wobei das Metall vorzugsweise aus der Gruppe, die aus Magnesium, Calcium, Strontium, Barium, Mangan, Eisen, Cobalt, Nickel, Kupfer, Zink, Silber, Cadmium, Bismut, Blei und Mischungen derselben besteht, ausgewählt wird, enthalten oder daraus bestehen.

8. Passives Kalt-Warm-Element nach einem der vorgenannten Ansprüche,
**dadurch gekennzeichnet,**
**dass** die anorganischen Partikel natürlich vorkommendes Calciumcarbonat, das vorzugsweise aus der Gruppe, die aus Calcit, Korallenkalk, Muschelkalk, Dolomit, Kalkstein, Kalkspat, Aragonit, Marmor, Travertin, Kreide und Mischungen davon besteht, ausgewählt wird und vorzugsweise einen Calciumcarbonat-Anteil, bezogen auf das Gesamtgewicht der anorganischen Partikel, aus einem Bereich von 50 bis 100 Gew.-% aufweist, enthalten oder daraus bestehen.

9. Passives Kalt-Warm-Element nach einem der vorgenannten Ansprüche,
**dadurch gekennzeichnet,**
**dass** die anorganischen Partikel Rügen-Kreide, vorzugsweise mit einem Calciumcarbonat-Anteil aus einem Bereich von 50 bis 100 Gew.-%, bevorzugt von 70 bis 100 Gew.-%, jeweils bezogen auf das Gesamtgewicht der anorganischen Partikel, enthalten oder daraus bestehen.

10. Passives Kalt-Warm-Element nach einem der vorgenannten Ansprüche,
**dadurch gekennzeichnet,**
**dass** die anorganischen Partikel präzipitiertes Calciumcarbonat, vorzugsweise mit einem Calciumcarbonat-Anteil, aus einem Bereich von 50 bis 100 Gew.-%, bevorzugt von 70 bis 100 Gew.-%, bezogen auf das Gesamtgewicht der anorganischen Partikel, enthalten oder daraus bestehen.

11. Passives Kalt-Warm-Element nach einem der vorgenannten Ansprüche,
**dadurch gekennzeichnet,**
**dass** die anorganischen Partikel eine Löslichkeit in Wasser bei 20°C von weniger als 2,0 g/l aufweisen.

12. Passives Kalt-Warm-Element nach einem der vorgenannten Ansprüche,
**dadurch gekennzeichnet,**
**dass** der Viskositätsregler thixotrope Eigenschaften aufweist.

13. Passives Kalt-Warm-Element nach einem der vorgenannten Ansprüche,
**dadurch gekennzeichnet,**
**dass** der Gelbildner und/oder Viskositätsregler bevorzugt aus der Gruppe, die aus Agar, Alginsäure, Alginate, Gummi arabicum, Traganth, Galactan, Carobgummi, Polyosen, Guarkernmehl, Karayagummi, Carrageen, Pektin, Quittensamenmehl, Casein, Gelatine, Kollagen, Albumin, Stärke, modifizierte Stärke, Tarakernmehl, Johannisbrotbaumkernmehl, Xanthan, Dextran, Gellan, Furcellaran, Pullulan, Phyllophoran, Chitin, Chitosan, Vinylpolymere, Polyoxyethylen-Polyoxypropylen-Copolymere, Polyacryl- und Polymethacrylverbindungen, Polyimine, Polyamide, Polycarbonsäuren, Polyoxyethylenpolymere, Polyether wie Polyethylenglycole, Cellulosepolymere wie Carboxymethylcellulose, Hydroxymethylcellulose, Hydroxypropylcellulose, Hydroxypropylmethylcellulose, Methylcellulose, Methylethylcellulose, Hyaluronsäure und Mischungen derselben besteht, ausgewählt wird.

14. Verwendung von anorganischen Partikeln mit einer spezifischen Dichte aus einem Bereich von 1,7 bis 7,0 und einer Partikelgröße D₅₀ von maximal 100 µm zur Herstellung eines passiven Kalt-Warm-Elementes.
